(19) Europäisches Patentamt
European Patent Office
Office européen des brevets

(11) Veröffentlichungsnummer: **0 036 159**
**B1**

# (12) EUROPÄISCHE PATENTSCHRIFT

(45) Veröffentlichungstag der Patentschrift: **27.06.84**

(21) Anmeldenummer: **81101713.6**

(22) Anmeldetag: **09.03.81**

(51) Int. Cl.³: **C 07 C 59/52,** C 07 C 59/64, C 07 C 69/732, C 07 C 69/734, C 07 C 51/353, C 07 C 67/343 // C08K5/10, C08K5/13

## (54) 4-(2,5-Dihydroxyphen-1-yl)-crotonsäure und deren Derivate sowie Verfahren zur Herstellung dieser Verbindungen.

(30) Priorität: **19.03.80 DE 3010474**

(43) Veröffentlichungstag der Anmeldung: **23.09.81 Patentblatt 81/38**

(45) Bekanntmachung des Hinweises auf die Patenterteilung: **27.06.84 Patentblatt 84/26**

(84) Benannte Vertragsstaaten: **BE CH DE FR GB IT LI NL**

(56) Entgegenhaltungen: **DE - A - 2 403 752**

(73) Patentinhaber: **BASF Aktiengesellschaft, Carl-Bosch-Strasse 38, D-6700 Ludwigshafen (DE)**

(72) Erfinder: **Horner, Michael, Dr., Im Ritterbueschel 9, D-6730 Neustadt (DE)**
Erfinder: **Nissen, Axel, Dr., Panoramastrasse 51, D-6906 Leimen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 036 159 B1

**Beschreibung**

Die vorliegende Erfindung betrifft die 4-(2,5-Dihydroxyphen-1-yl)-crotonsäure und deren Derivate der allgemeinen Formel I

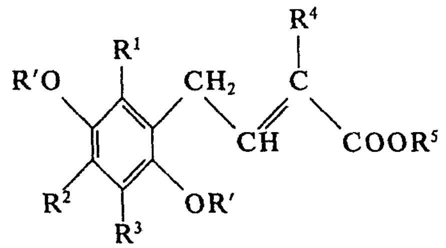

(I)

Hierin haben die Substituenten folgende Bedeutung:

$R^1-R^4$ H; $C_1-C_8$-Alkyl
$R^5$ H; $C_1-C_{30}$-Alkyl; $C_2-C_{30}$-Alkenyl
R′ H; $C_1-C_8$-Alkyl oder Acyl; Benzyl oder durch inerte Substituenten substituiertes Benzyl

Außerdem betrifft die Erfindung die Herstellung dieser Verbindungen.

Es ist allgemein bekannt, daß sich Verbindungen, die in ihrem Molekül eine Hydrochinonstruktur enthalten, als Stabilisatoren von organischen Massen, vor allem von Kunststoffen, gegen die Einflüsse von Licht, Wärme und Oxidationsmitteln eignen. Solche Verbindungen sind z. B. das $\alpha$-Tocopherol

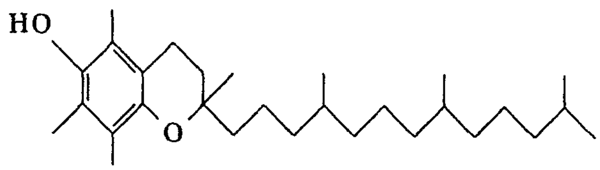

und damit strukturverwandte Chromanderivate.

Der Erfindung lagen neue Hydrochinonderivate als Aufgabe zugrunde, die selber als Stabilisatoren verwendet werden können oder die sich zur Herstellung von derartigen Stabilisatoren eignen und die ganz allgemein die Synthesemöglichkeiten auf diesem Gebiet erweitern.

Besondere Bedeutung kommt hierbei der Herstellung der Chromanderivate IV

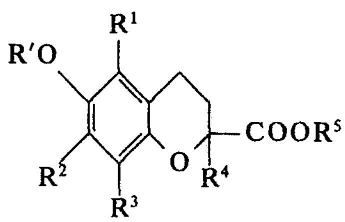

(IV)

zu, die bisher nur in einer vielstufigen Synthese unter Verwendung hochgiftiger Cyanide oder auf dem mit unzureichenden Ausbeuten (rd. 15% Reinprodukt) verbundenen Wege über eine Mannichreaktion zugänglich sind (US-A-3 947 473).

Demgemäß wurden die eingangs definierten neuen Verbindungen gefunden, die man erhält, wenn man ein Hydrochinon II

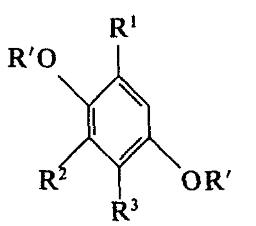

(II)

in Gegenwart von 0,1 bis 2 mol, pro Mol II einer Lewissäure

a) mit einem Vinylglycolsäurederivat IIIa

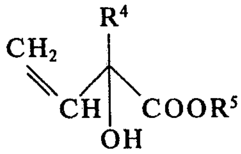

(IIIa)

oder

b) mit einem Crotonsäurederivat IIIb

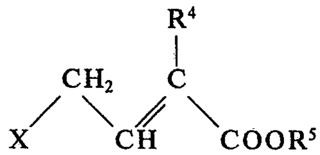

(IIIb)

wobei X Halogen, die Hydroxylgruppe oder eine $C_1-C_4$-Alkoxy- oder Acyloxygruppe bedeutet umsetzt.

Als Lewissäuren eignen sich beispielsweise $BF_3$, $AlCl_3$, $ZnBr_2$, $ZnJ_2$ und vor allem $ZnCl_2$, und zwar vorzugsweise in Mengen von 0,2 bis 1 mol pro Mol II. Besonders vorteilhaft ist die Mitverwendung von 1 bis 5 mol einer starken wasserfreien Mineralsäure pro Mol der Lewissäure. Solche Säuren sind vor allem HCl und HBr.

Vorzugsweise setzt man die Verbindungen II und IIIa bzw. IIIb im äquimolaren Mengenverhältnis miteinander um, jedoch kann es auch zweckmäßig sein, IIIa bzw. IIIb in einem bis zu 1,5molaren Überschuß einzusetzen.

Es empfiehlt sich, beide Verfahrensvarianten (a) und (b) in Gegenwart eines inerten aprotischen Lösungsmittels vorzunehmen, dessen Donizität höchstens 20 beträgt. Die Donizität, die in »Angew. Chemie«, Band 82 (1972), S. 858, definiert ist, ist eine Maßzahl für die Stabilität von Komplexverbindungen aus Lewissäuren und Lösungsmitteln. Demgemäß eignen sich schwach komplexbildende Lösungsmittel wie Benzol, Chlorbenzol, Nitromethan, Acetonitril, Dichlormethan, 1,2-Dichloräthan, Chloroform und vor allem n-Hexan, n-Heptan und Toluol. Die Funktion der Lösungsmittel besteht darin, mindestens einen Teil der Reaktionspartner in Lösung zu halten.

Hieraus ergibt sich die Menge des Lösungsmittels, die in weiten Grenzen schwanken kann, jedoch im allgemeinen 5 bis 15 kg pro Kilogramm II beträgt.

Die Reaktionstemperatur liegt vorzugsweise zwischen 30 und 150°C, insbesondere zwischen 60 und 130°C. Da die Reaktion bei Normaldruck ausgeführt werden kann, ist das Arbeiten bei vermindertem oder erhöhtem Druck im allgemeinen nicht erforderlich.

Die Reaktionsführung erfolgt nach den üblichen präparativen Techniken der Friedel-Craffts-Reaktion, indem man etwa ein Gemisch aus II und der Lewissäure vorlegt und hierzu bei der Reaktionstemperatur allmählich IIIa bzw. IIIb gibt. Verwendet man eine starke Mineralsäure mit, so legt man etwa die Hälfte dieser Säure ebenfalls vor und dosiert die andere Hälfte im Laufe der Reaktion zu. In allen Fällen ist es zweckmäßig, das Reaktionsgemisch intensiv zu rühren und wegen der Oxidationsempfindlichkeit der Reaktionspartner unter Luftausschluß zu arbeiten.

Nach beendeter Reaktion, die normalerweise 3 bis 10 Stunden in Anspruch nimmt, arbeitet man das Reaktionsgemisch wie üblich auf, indem man das Gemisch mit Wasser versetzt und das Verfahrensprodukt I aus der organischen Phase isoliert. Die Ausbeuten an I liegen im allgemeinen zwischen 50 und 75%.

Unter den Verfahrensprodukten I haben diejenigen die größte Bedeutung, in denen die Reste $R^1$ bis $R^4$ für Methylgruppen stehen.

Sollen die Verbindungen I selber als Stabilisatoren dienen, so ist der Rest $R^5$ vorzugsweise eine $C_{10}-C_{30}$-Alkyl- oder Alkenylgruppe.

Als Zwischenprodukte kommen hingegen vornehmlich solche in Betracht, in denen $R^5$ Wasserstoff bedeutet.

Soweit R' nicht für Wasserstoff, sondern für eine der definitionsgemäßen Schutzgruppen steht, kommen hierfür vor allem die Methylgruppe, die tert.-Butylgruppe sowie die Benzylgruppe in Betracht, da diese Gruppen sich hydrolytisch oder hydrogenolytisch besonders leicht wieder abspalten lassen. Allgemein kann R' neben Wasserstoff vorzugsweise eine $C_1-C_8$-Alkyl- oder Acylgruppe oder eine gegebenenfalls durch inerte Substituenten substituierte Benzylgruppe sein.

Die den Verbindungen I zugrunde liegenden Ausgangsverbindungen II, IIIa und IIIb sind bekannt oder nach bekannten Methoden erhältlich.

Die Verbindungen I lassen sich in Chromanderivate überführen, wie an folgendem Beispiel veranschaulicht sei:

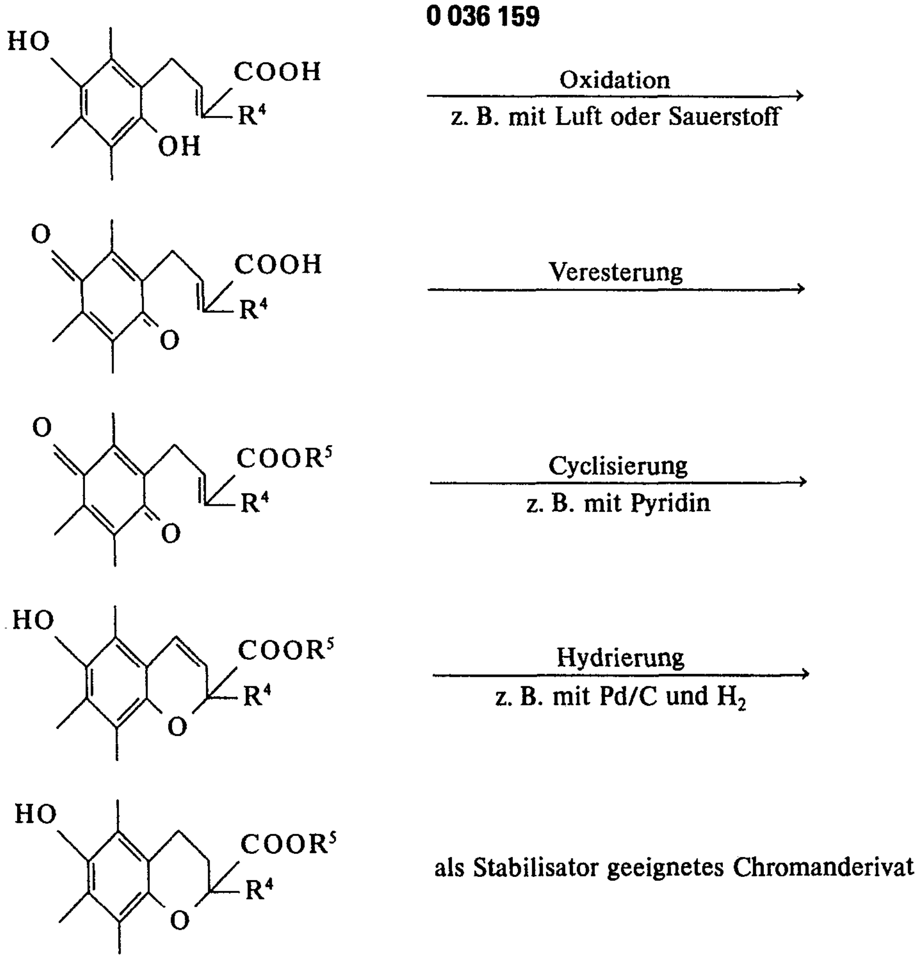


## Beispiel 1

4-(2,5-Dihydroxy-3,4,6-trimethylphen-1-yl)-2-methylcrotonsäure, hergestellt nach Methode (a)

Ein Gemisch aus 24 g (0,2 mol) Vinylmilchsäure und 10 g 0,07 mol) wasserfreiem $ZnCl_2$ wurde unter intensivem Rühren und unter Luftausschluß zum Sieden erhitzt. Danach wurde innerhalb von 15 min 1 l wasserfreier Chlorwasserstoff eingeleitet und innerhalb weiterer 45 min 30,4 g (0,2 mol) Trimethylhydrochinon hinzugegeben. Nachdem nochmals 200 ml HCl-Gas eingeleitet worden waren, wurde das Gemisch zur Vervollständigung der Umsetzung noch 4 h unter Rückflußkühlung erhitzt. Die übliche Aufarbeitung lieferte das oben genannte Verfahrensprodukt in 65%iger Ausbeute; Smp. 208° C (Zers.).

## Beispiel 2

4-(2,5-Dihydroxy-3,4,6-trimethylphen-1-yl)-2-methylcarbonsäure, hergestellt nach Methode (b)

In ein auf 98° C erhitztes Gemisch aus 9,0 g (0,06 mol) Trimethylhydrochinon, 8,0 g (0,06 mol) 4-Chlor-2-methylcrotonsäure, 3 g (0,02 mol) wasserfreiem $ZnCl_2$ und 80 ml Heptan wurden in Laufe einer Stunde 3 l HCl-Gas eingeleitet. Nach zweistündigem Rühren wurde die Heptanphase dekantiert und der verbleibende ölige Rückstand 24 h bei 20° C mit Methanol gerührt. Hierbei kristallisierte ein Teil des Verfahrensproduktes aus. Der restliche Teil wurde mit Wasser aus der methanolischen Phase ausgefällt. Kristallisat und Niederschlag wurden vereinigt und aus Wasser umkristallisiert. Die Ausbeute an dem oben genannten Produkt betrug 62%.

## Beispiel 3

4-(2,5-Dihydroxy-3,4,6-trimethylphen-1-yl)-2-methylcrotonsäuremethylester

In eine Lösung aus 34 ml Nitromethan und 250 ml Toluol wurden unter Rühren bei 0° C im Laufe von 45 min zunächst mit 23 g (0,34 mol) $BF_3$ eingeleitet. Danach wurde die Lösung mit 51,4 g (0,34 mol) 2,3,5-Trimethylhydrochinon und anschließend im Laufe von 3 h mit 44,2 g (0,34 mol) Vinylmilchsäuremethylester versetzt. Nach weiterem 10stündigem Rühren bei 15° C wurde das Reaktionsgemisch wie üblich aufgearbeitet, wobei das oben genannte Verfahrensprodukt in 20%iger Ausbeute ausfiel; Smp.

109—111°C.

Beispiel 4

4-(2,5-Dihydroxy-3,4,6-trimethylphen-1-yl)-2-methylcrotonsäure-n-heptylester

Analog Beispiel 3, jedoch ausgehend vom Vinylmilchsäure-n-heptylester, wurde die oben genannte Verbindung in 15%iger Ausbeute hergestellt; Smp. 39—42°C.

**Patentansprüche**

1. 4-(2,5-Dihydroxyphen-1-yl)-crotonsäure und deren Derivate der allgemeinen Formel I

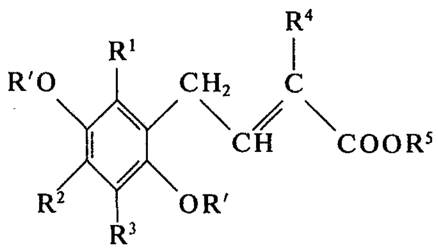

(I)

in der die Substituenten folgende Bedeutung haben:

| | |
|---|---|
| $R^1$—$R^4$ | H; $C_1$—$C_8$-Alkyl |
| $R^5$ | H; $C_1$—$C_{30}$-Alkyl;<br>$C_2$—$C_{30}$-Alkenyl |
| R′ | H; $C_1$—$C_8$-Alkyl oder Acyl; Benzyl oder durch inerte Substituenten substituiertes Benzyl |

2. Verfahren zur Herstellung der Verbindung I, dadurch gekennzeichnet, daß man ein Hydrochinon II

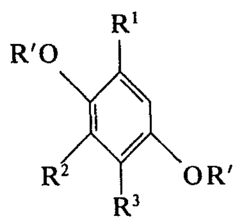

(II)

in Gegenwart von 0,1 bis 2 mol, pro Mol II einer Lewissäure

a) mit einem Vinylglycolsäurederivat IIIa

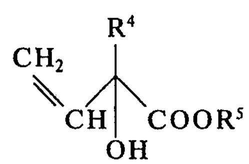

(IIIa)

oder

b) mit einem Crotonsäurederivat IIIb

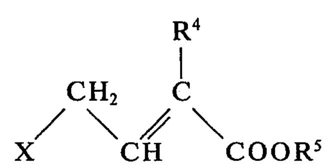

(IIIb)

wobei X Halogen, die Hydroxylgruppe oder eine $C_1$—$C_4$-Alkoxy- oder Acyloxygruppe bedeutet umsetzt.

**Claims**

1. 4-(2,5-Dihydroxyphen-1-yl)-crotonic acid and its derivatives of the general formula I

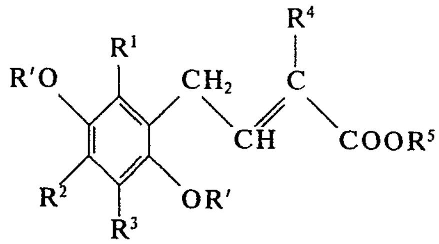

(I)

where $R^1$, $R^2$, $R^3$ and $R^4$ are each H or $C_1-C_8$-alkyl, $R^5$ is H, $C_1-C_{30}$-alkyl or $C_2-C_{30}$-alkenyl, and R′ is H, $C_1-C_8$-alkyl or acyl, or benzyl optionally substituted by inert substituents.

2. A process for the preparation of a compound I, wherein a hydroquinone II

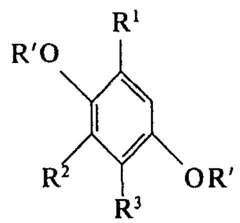

(II)

is reacted, in the presence of from 0.1 to 2 moles of a Lewis acid per mole of II, with

a) a vinylglycolic acid derivative IIIa

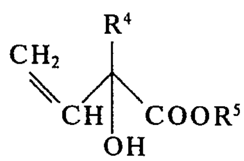

(IIIa)

or

b) a crotonic acid derivative IIIb

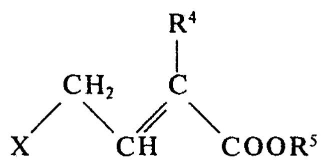

(IIIb)

where X is halogen, hydroxyl or $C_1-C_4$-alkoxy or acyloxy.

**Revendications**

1. L'acide 4-(2,5-dihydroxyphényl-1)-crotonique et ses dérivés de formule générale I

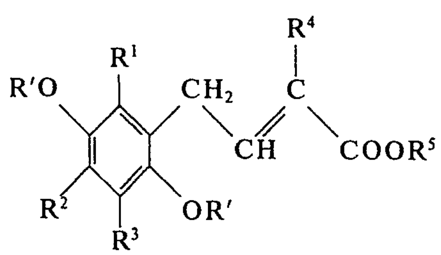

(I)

dans laquelle les symboles ont les significations suivantes:

| | |
|---|---|
| $R^1$ à $R^4$ | H; alkyle en C1—C8 |
| $R^5$ | H; alkyle en C1—C30; alcényle en C2—C30 |
| R′ | H; alkyle en C1—C8 ou acyle; benzyle ou benzyle portant des substituants inertes. |

2. Procédé de préparation des composés I, caractérisé en ce que l'on fait réagir une hydroquinone II

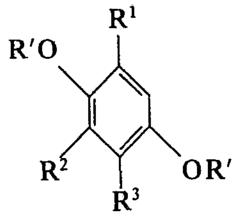

(II)

en présence de 0,1 à 2 moles, par mole du composé II, d'un acide Lewis

a) avec un dérivé d'acide vinylglycolique IIIa

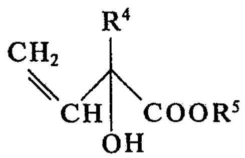

(IIIa)

ou bien

b) avec un dérivé de l'acide crotonique IIIb

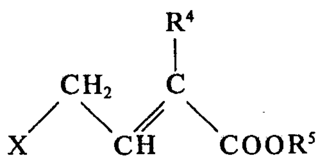

(IIIb)

dans lequel X représente un halogène, le groupe hydroxy ou un groupe alcoxy en C1—C4 ou alcyloxy.